Europäisches Patentamt

European Patent Office

Office européen des brevets

(19)

(11) Publication number: **0 133 046**
**A2**

# EUROPEAN PATENT APPLICATION

(12)

(21) Application number: 84305178.0

(22) Date of filing: 30.07.84

(51) Int. Cl.⁴: **C 12 N 15/00**
**A 23 C 9/123**

(30) Priority: 30.07.83 JP 140257/83

(43) Date of publication of application:
13.02.85 Bulletin 85/7

(84) Designated Contracting States:
DE FR GB NL SE

(71) Applicant: KABUSHIKI KAISHA YAKULT HONSHA
1-19, Higashishinbashi 1-chome
Minato-ku Tokyo 105(JP)

(72) Inventor: Kadota, Mariko
15-22, 2-chome Nakamachi
Koganei Tokyo(JP)

(72) Inventor: Kudo, Satoshi
78-3, Shimoyotsugi
Akigawa Tokyo(JP)

(72) Inventor: Mutai, Masahiko
988, 4-chome Shimizu
Higashiyamato Tokyo(JP)

(74) Representative: Brewer, Leonard Stuart et al,
Sanderson & Co. 97 High Street
Colchester Essex CO1 1TH(GB)

(54) A method of introducing a donor DNA into a strain of the genus Lactobacillus.

(57) A method of introducing a donor DNA into a strain belonging to the genus *Lactobacillus*, comprises preparing spheroplasts of the strain, encapsulating donor DNA within liposomes to obtain DNA-encapsulating-liposomes, and allowing the DNA-encapsulating-liposomes to fuse with the spheroplasts in the presence of a fusion inducing agent such as polyethylene glycol. The donor DNA may be provided in the form of recombinant DNA molecules prepared as a vector having inserted therein suitable phage DNA, plasmid DNA or heterogenic DNA retaining useful genetic information. Examples of the phage DNA include the DNA of the virulent phage J1 and the DNA of the temperate phage $\phi$FSW selectively retained in the genus *L. casei*.

## A METHOD OF INTRODUCING A DONOR DNA INTO
## A STRAIN OF THE GENUS LACTOBACILLUS

The present invention relates to a method of introducing a donor deoxyribonucleic acid into a microorganism, particularly into a strain belonging to the genus Lactobacillus, using a liposome within which the donor deoxyribonucleic acid (hereinafter referred to as DNA) is encapsulated.

Various attempts have thus far been made in quest of genetic engineering techniques to have donor DNA's introduced into host microorganisms. These techniques are basically broken down to three major categories. One method is to use competent cells as the host and to have a donor DNA introduced directly into the host cells, and another method is to also use competent cells as the host and to have the host cells fused with liposomes having a donor DNA encapsulated therein. The third method is to use cells in the form of spheroplasts as the host cells and to have a donor DNA introduced directly into the host cells.

Each of these methods is applicable only to limited types of microorganisms. Where it is desired to use cells of a strain of the genus Lactobacillus as the host cells in particular, difficulties have been encountered in introducing a donor DNA into the cells of the particular strain. Thus, there have been no reports evidencing success in introducing a donor DNA into cells of a culture belonging to the genus Lactobacillus.

One of the major reasons which account for the difficulties of introducing a donor DNA into a culture of the genus Lactobacillus is that the state of the competent cells of a strain of the genus is not known. For this reason, the first two of the above mentioned three methods are not operable for the introduction of a donor DNA into the competent cells of a strain belonging to the genus Lactobacillus. Another reason is that it has been deemed difficult to prepare spheroplast cells of a strain of the genus Lactobacillus, forming a bar to an approach toward the adoption of the third method. A third barrier to introduction of a donor DNA into a strain of the genus Lactobacillus results from the property of the strain by which it secretes a large quantity of deoxyribonuclease which destroys the donor DNA to be introduced into the strain of the genus Lactobacillus.

For these reasons which are intrinsic in strains of the genus Lactobacillus, it has been considered difficult to have a donor DNA introduced directly or with the aid of liposomes into a strain belonging to the genus Lactobacillus. Development of a technique to permit introduction of a donor DNA into a strain of the genus Lactobacillus, however, would be very useful       in the related field of industry particularly in the art of manufacturing fermented milk products.

It is, accordingly, an important object of the present invention to provide a method of introducing a donor DNA into a strain belonging to the genus Lactobacillus.

It is another important object of the present invention to provide a method of introducing into a strain belonging to the genus Lactobacillus a bacteriophage as a donor DNA which has been trapped in a liposome.

In accordance with the present invention, there is provided a method of introducing a donor DNA into a strain belonging to the genus <u>Lactobacillus</u>, comprising preparing spheroplasts of the strain, encapsulating donor DNA within liposomes to obtain DNA-encapsulating-liposomes, and allowing the DNA-encapsulating-liposomes to fuse with the spheroplasts in the presence of a fusion inducing agent.  It should    be borne in mind that the "spheroplasts" herein referred to include not only    spheroplasts in the strict sense of the term but also protoplasts of any strain of the genus <u>Lactobacillus</u>.

The spheroplasts of the strain of the genus <u>Lactobacillus</u> used as the host can be prepared advantageously by the method of Tomochika et al (K. Tomochika, M. Funabashi, A. Nagata, T. Fujii and Y. Kanemasa, <u>Jpn. J. Bacteriol.</u>, Vol. 37, pages 777 to 785, 1982).

Encapsulation of the donor DNA within the liposomes may be effected by the reverse-phase evaporation method (F. Szoka, Jr. and D. Papahadjopoulos, <u>Proc. Natl. Acad. Sci. (U.S.A.)</u>, Vol. 75, page 4194, 1978) using egg-yolk lecithin.

The fusion between the DNA-encapsulating-liposomes and the spheroplasts is effected in the presence of, for example, polyethylene glycol and as a result of the fusion therebetween, the donor DNA trapped in the liposomes is introduced into the spheroplast cells of the genus <u>Lactobacillus</u>. Preferred examples of the polyethylene glycol used as the fusion inducing agent include polyethylene glycol 4,000 and polyethylene glycol 1,000, although other types of fusion inducing agent may be utilized.

The product of fusion, viz. the spheroplasts fused with the liposomes having the donor DNA encapsulated therein, may be cultured in a suitable hyper medium so that the genetic information retained in the donor DNA is expressed within the spheroplast cells of the strain of the genus Lactobacillus.

The donor DNA to be used in the method according to the present invention may be provided in the form of recombinant DNA molecules prepared as a vector having inserted therein suitable phage DNA, plasmid DNA or heterogenic DNA, retaining desired genetic information. In this instance, it is advisable that the recombinant DNA molecules be selected to have a relatively small molecular weight which is easy to be trapped within liposomes although DNA molecules with a molecular weight of the order of $2.7 \times 10^7$ are operable. Preferred examples of the phage DNA include the DNA of the virulent phage J1 and the temperate phage øFSW selectively retained in the species Lactobacillus casei although any other types of phage DNA's are operable for carrying out the present invention.

The transformant obtained by introducing the donor DNA into the spheroplast cells of the Lactobacillus may be detected by a method which is ordinarily used for the detection of a transformant in the conventional genetic engineering techniques using, for example, a donor DNA having a suitable genetic marker retained therein.

The invention is illustrated by the following Examples.


Example 1

- 5 -                    0133046

## 1. Preparation of Spheroplasts

Strain YIT9031 of the species L. casei was used as the host in Example 1. The host strain was cultured in 250 milliliters of modified "de Vries" medium consisting, per liter, of the following constituents:

| | |
|---|---|
| Nutrient broth: | 12 grams |
| Yeast extract: | 5 grams |
| Tween 80[(*)]: | 1 milliliter |
| $K_2HPO_4$: | 3 grams |
| $KH_2PO_4$: | 1.5 gram |
| Glucose: | 0.4 gram |
| $MgSO_4.7H_2O$: | 0.1 gram |
| $MnSO_4.4H_2O$: | 0.025 gram |

(*) RTM (Atlas Powder Company, U.S.A.)

Of these constituents, glucose, $MgSO_4.7H_2O$ and $MnSO_4.4H_2O$ were respectively sterilized in different autoclaves and were added in the above mentioned proportions to the other constituents. The modified "de Vries" medium thus prepared was adjusted to a final pH value of 6.8.

The cultivation was carried out at 37°C under static conditions. The growth of the cells of the strain YIT9031 was continuously monitored through detection of the variation in the optical density (herein denoted as $OD_{650}$) of the clump at the wavelength of 650 nanometers until the $OD_{650}$ readings reached a value indicating that $8 \times 10^8$ cells were grown per milliliter of the culture medium. The cells thus grown in the modified "de Vries" medium were harvested, washed three times each in 30 milliliters of 20 mM potassium phosphate buffer (pH: 5.8) and suspended in a 20 mM potassium phosphate buffer containing an additive of 30 milliliters of 1 M sucrose to an optical density $OD_{650}$ of 0.3. To the resultant suspension was added N-acetylmuramidase M1 (commercially available as "N-Acetylmuramidase SG" from Dainippon Pharmaceutical Co., Ltd., Osaka, Japan) in a quantity which resulted in a final concentration of 3 micrograms per milliliter. The preparation thus obtained was gently shaken at 37°C for 20 minutes to incubate the cells therein and was thereafter subjected to centrifugation at 9000 x g for 15 minutes. A pellet was obtained by removing the supernatant of the resultant preparation by sucking the supernatant. The pellet was suspended, by shaking, in 0.5 milliliter of 20 mM potassium phosphate buffer (pH: 5.8) containing 1 percent (W/V) bovine serum albumin, 10 percent (V/V) equine serum and 1 M sucrose, resulting in a suspension containing steroplasts of the strain YIT9031 in a density of $10^9$ cells per milliliter. As to the N-acetylmuramidase M1 herein used, reference may be made to K. Yokogawa, S. Kawata, T. Takemura and Y. Yoshimura, <u>Agric. Biol. Chem</u>, Vol. 37, pages 799-808, 1973, Tokyo.

2. <u>Preparation of Liposome Encapsulating DNA</u>

A donor DNA was prepared using the virulent phage J1 peculiar to the species <u>L</u>. <u>casei</u> by the method taught by Khosaka (T. Khosaka, <u>J. Gen. Virol.</u>, Vol. 37, pages 209-214, 1977). For this purpose, the J1 phage DNA was transformed into the strain <u>L</u>. <u>casei</u> YIT9031 and was therein incubated to cause lysis of the host cells. The resultant lysate was treated with deoxyribonuclease (which may be substituted by ribonuclease) to decompose the free nucleic acids contained in the lysate. The preparation thus obtained was mixed with polyethylene glycol to condense the phage particles therein. The condensed phage particles were purified by the known cesium-chloride density-gradient centrifugation method followed by a phenol extraction process, thereby producing a solution containing the Jl phage DNA.

Liposomes having the Jl phage DNA trapped therein were prepared by the known reverse-phase evaporation method (F. Szoka, Jr. and D. Papahadjopoulos, Proc. Natl. Acad. Sci. (U.S.A.), Vol. 75, page 4194, 1978). For this purpose, 64.5 milligrams of egg-yolk lecithin and 21.5 milligrams of egg-yolk lecithin partially hydrolyzed with phospholipase D were sufficiently dried under a subatmospheric pressure of 1 mm of Hg and were thereupon dissolved in 3 milliliters of diethylether. The details of the latter egg-yolk lecithin are disclosed in Phospholipids, pages 92-93, Elsevier Publishing Co., 1964, New York. To the resultant solution were added (1) 0.6 milliliter of 5 mM N-2-hydroxyethyl piperazine-N'-2-ethane sulfonic acid (HEPES) buffer containing 50 mM NaCl, 50 mM KCl and 0.1 mM sodium ethylenediaminetetraacetic acid (EDTA) and (2) 0.4 milliliter of DNA buffer (pH: 7.4) consisting of 10 mM Tris-HCl and 0.1 mM EDTA in addition to 82 micrograms of the phage DNA-containing solution produced as described above. The mixture of these was, upon vortical shaking, subjected to sonication for 15 seconds at 0°C, whereupon ether was removed from the sonicated mixture at 25°C under a subatmospheric pressure of 400 mm of Hg by rotary evaporation at 200 rpm. In approximately 8 minutes after the evaporation process, 0.5 milliliter of the aforesaid HEPES buffer was added to the resultant preparation, which was then subjected, upon shaking, to a second evaporation process at 25°C, 700 mm of Hg and 200 rpm for more than 30 minutes to thoroughly remove the residual ether therefrom.

The crude liposome suspension obtained in this way was diluted with 1 milliliter of 0.4 mM mannitol (T. Nagata, Kagaku to Seibutsu (Chemistry and Biology), Vol. 20, pages 177-184, 1982, Tokyo) and the resultant solution was continuously filtered through Uni-Pore filters with the pore sizes of 3 and 4 micrometers (F. Olson, C.A. Hunt, F.C. Szoka, W.J. Vail and D. Papahadjopoulos, Biochim. Biophys. Acta., Vol. 557, page 9, 1979). Liposomes having the J1 phage DNA encapsulated therein were thus obtained in the form of a suspension.

3. Fusion of Steroplast Cells and Liposomes

Five microliters of this liposome suspension was mixed with 55 microliters of the fresh suspension of the spheroplasts of the strain YIT9031 prepared as described above. The mixture was preincubated at 37°C for 30 minutes, followed by addition thereto of 140 microliters out of a total of 28 milliliters of polyethylene glycol solution consisting of 20 grams of polyethylene glycol 4,000, 1.4 milliliter of 20 percent (W/V) bovine serum albumin, 2.8 milliliter of equine serum and 5.8 milliliter of 4 mM HEPES (pH: 7.4). The mixture was incubated at 37°C for 2 minutes to cause fusion between the spheroplast cells and the liposomes having the J1 phage DNA encapsulated therein.

4.  Detection of J1 Phage DNA Introduced Cells

The soft agar method for phage detection (Mark H. Adams, Bacteriophage, pages 454-456, Wiley Interscience, New York) was employed to detect the host spheroplast cells into which the J1 phage DNA was introduced.

For this purpose, 200 microliters of the mixed solution of the spheroplast cells fused with the liposomes obtained as described above was added to 1.8 milliliters of MRT phage growth medium (A. Murata, Agric. Biol. Chem., Vol. 35, page 667) consisting of 1 M sucrose, 1 percent (W/V) bovine serum albumin, 10 percent (W/V) equine serum and 20 mM Tris-maleate buffer (pH: 6.4) in addition to, for 1 liter of the growth medium, the following constituents:

| | |
|---|---|
| Polypeptone[**]: | 10 grams |
| Glucose: | 10 grams |
| Sodium acetate: | 10 grams |
| Yeast extract: | 3 grams |
| Meat extract: | 3 grams |
| NaCl: | 1.5 grams |
| $MgCl_2$: | 1 gram |
| $MgSO_4 \cdot 7H_2O$: | 0.2 gram |
| $MnSO_4 \cdot H_2O$: | 10 milligrams |
| $FeSO_4 \cdot 7H_2O$: | 1 milligram |

[**] RTM.

The resultant mixture was further incubated at 37°C for 3 hours to permit gene expression of the J1 phage DNA introduced into the host DNA. To 1 milliliter of the mixture thus prepared were added 5 milliliter of L. casei strain YIT9031 solution containing approximately $10^9$ cells per milliliter and 9 milliliters of 0.6 percent (W/V) MRT soft agar solution containing 0.5 M sucrose. The product was incubated on an MRT agar stub at 37°C for 24 hours.

The soft agar mixture obtained in this way was applied on an MRT agar stub and was assayed by counting the plaques created on the stub as a result of the incubation, revealing that the plaque-forming units (PFU) on the soft agar mixture was 150 per milliliter of the mixture. This means that the frequency at which the J1 phage DNA is capable of transforming the host spheroplasts and expressing the genetic information thereof is $7 \times 10^{-8}$ per molecule of the J1 phage DNA, in consideration of the fact that the 1 milliliter of the soft agar mixture contains 2.5 microliters of liposome solution (which contains 0.1 microgram or less of DNA) and that the molecular weight of the J1 phage DNA is $2.7 \times 10^7$.

Example 2

Strain YIT9031 of the species L. casei was again used as the host and was processed into spheroplast cells in a way similar to that of Example 1. A donor DNA was prepared using the temperate phage øFSW (Appl. Environ. Microbial., Vol. 39, pages 472-476) of the species L. casei and liposomes having the øFSW phage DNA trapped therein were prepared in a way essentially similar to that used in Example 1. The øFSW phage DNA successfully transformed the spheroplast cells of the strain YIT9031, causing lysis of the host spheroplast cells. The resultant lysate was treated with deoxyribonuclease or ribonuclease and the preparation thus obtained was mixed with polyethylene glycol to condense the phage particles therein and remove the free nucleic acids contained in the lysate. The condensed phage particles were purified by the cesium-chloride density-gradient centrifugation method followed by a phenol extraction process, thereby producing a solution containing the øFSW phage DNA. A soft agar mixture was prepared from this solution in a way  similar to that used in Example 1 and was assayed, also by the soft agar method, for the number of the spheroplast cells which were transformed with the øFSW phage DNA. The results of the assays showed that the øFSW phage DNA is also capable of transforming the host spheroplasts of the L. casei strain YIT9031 to express the genetic information thereof at a frequency comparable with the frequency mentioned in Example 1 in which the Jl phage DNA was used as the donor DNA.

While the strain of the genus Lactobacillus used as the host has been exemplified by the strain YIT9031 of the species L. casei in each of Examples 1 and 2 above, this is simply for the purpose of description and any of the other strains of the particular species or any of the strains belonging to any of the other species of the genus Lactobacillus may be used in lieu of the strain YIT9031. Similarly, the Jl phage DNA used as the donor DNA in Example 1 or the øFSW phage DNA used as the donor DNA in Example 2 may be replaced with any other type of phage DNA or with any type of plasmid DNA or heterogenic DNA.

As will have been appreciated from the foregoing description, the method according to the present invention is applicable to recombinant DNA molecules prepared as a suitable vector which retains a DNA having useful genetic information thereon and is adapted to have the useful genetic information expressed within the cells of a strain belonging the genus Lactobacillus. Thus, the method according to the present invention is expected to provide potent tools for the breeding and improvement of cells of strains belonging to the genus Lactobacillus and for unveiling the mechanism through which the genetic information retained by a strain of the genus Lactobacillus is to be expressed.

0133046

## CLAIMS

1. A method of introducing a donor DNA into a strain belonging to the genus Lactobacillus, comprising preparing spheroplasts of the strain, encapsulating the donor DNA within liposomes to obtain DNA-encapsulating- -liposomes, and allowing the DNA-encapsulating-liposomes to fuse with the spheroplasts in the presence of a fusion inducing agent.

2. A method according to claim 1 in which the fusion inducing agent is polyethylene glycol.

3. A method according to claim 1 or 2 in which the donor DNA is recombinant DNA prepared as a vector having inserted therein bacteriophage DNA.

4. A method according to claim 1 or 2 in which the donor DNA is recombinant DNA prepared as a vector having inserted therein plasmid DNA.

5. A method according to claim 3 in which the bacteriophage is the phage J1 peculiar to the species L. casei.

6. A method according to claim 3 in which the bacteriophage is the phage øFSW peculiar to the species L. casei.

7. A method according to claim 3 or 4 in which the recombinant DNA has a molecular weight of up to about $2.7 \times 10^7$.

8. A method according to any one of the preceding claims in which the strain belongs to the species L. casei.

9. A method according to any one of the preceding claims in which the spheroplasts are protoplasts.

10. A method for the manufacture of fermented milk products employing a strain belonging to the genus Lactobacillus into which a donor DNA has been introduced by a method claimed in any one of the preceding claims.